(19) Europäisches Patentamt
European Patent Office
Office européen des brevets

(11) EP 1 229 124 A1

(12) **EUROPEAN PATENT APPLICATION**
published in accordance with Art. 158(3) EPC

(43) Date of publication:
**07.08.2002 Bulletin 2002/32**

(21) Application number: **00969954.7**

(22) Date of filing: **20.10.2000**

(51) Int Cl.⁷: **C12N 15/867**, C12N 15/90

(86) International application number:
**PCT/JP00/07337**

(87) International publication number:
**WO 01/29244 (26.04.2001 Gazette 2001/17)**

(84) Designated Contracting States:
**AT BE CH CY DE DK ES FI FR GB GR IE IT LI LU MC NL PT SE**

(30) Priority: **20.10.1999 JP 29843399**

(71) Applicant: **HISAMITSU PHARMACEUTICAL CO. INC.**
**Tosu-shi, Saga-ken 841-0017 (JP)**

(72) Inventors:
- **GOTO, Takeshi,**
**Hisamitsu Pharmaceutical Co.,Inc.**
**Tsukuba-shi, Ibaraki 305--0856 (JP)**
- **IIJIMA, Osamu,**
**Hisamitsu Pharmaceutical Co.,Inc.**
**Tsukuba-shi, Ibaraki 305-0856 (JP)**
- **SHIMADA, Takashi**
**Tokyo 113-0023 (JP)**

(74) Representative: **Grünecker, Kinkeldey,**
**Stockmair & Schwanhäusser Anwaltssozietät**
**Maximilianstrasse 58**
**80538 München (DE)**

(54) **NUCLEIC ACID TRANSPORTER CONTAINING POLYPEPTIDE AND RECOMBINANT VIRUS VECTOR**

(57) The nucleic acid carrier comprising a polypeptide comprising diaminobutyric acid and/or a pharmaceutically acceptable salt thereof and a recombinant viral vector (preferably containing a therapeutic gene) is transferred into cells efficiently and safely to enable high expression of the therapeutic gene in the cells whereby gene therapy is performed.

EP 1 229 124 A1

## Description

### Technical Field

This invention relates to nucleic acid carriers containing recombinant viral vectors for use in gene therapy.

### Background Art

In the clinical applications of gene therapy, the development of methods for and the forms of genes optimal for the efficient and safe transfer of therapeutic genes into target cells have proved to be one of the major technical tasks.

In early 1980s physical techniques such as microinjection were applied for use, but they were not able to effect the consistent and efficient transfer of therapeutic genes required for the treatment of diseases and their clinical applications were not realized. Later, recombinant viral vectors were developed that serve as vehicles for the efficient transfer of the therapeutic genes into cells; and this has, for the first time, allowed the clinical applications of gene therapy.

In the United States two hundred twelve cases of gene therapy protocols were approved by the Recombinant DNA Committee in the NIH up till April of 1998, and clinical trials were already conducted on over 2,000 patients against genetic diseases such as chronic immunodeficiency, familial hypercholesterolemia, and cystic fibrosis and various cancers (Hum. Gene Therapy, 9:2143-2161, 1998).

It is impossible to transfer therapeutic genes into all the target cells by using recombinant viral vectors clinically used at present. Thus, in the methods of treatment to which blood cells are subjected therapy is conducted such that after the target cells are separated from the body, they undergo gene transfer *in vitro* and then returned to the body again ("ex vivo gene transfer" in U.S. Pat. No. 5,399,346). This technique, however, experiences low efficiency in gene transfer (Dunbar, C. E., et al., Blood 85, 3048-3057, 1995) and requires multiple operations of gene transfer: a problem still remains in the improvement of efficiency of gene transfer.

In the method of improving the efficiency of gene transfer for such recombinant viral vectors, attempts are being made to efficiently transfer genes into the objective cells by allowing substances capable of forming complexes with viral vectors to interact with virus. Specifically mentioned is a method utilizing as the vehicle that should form a complex with a recombinant viral vector, the following: polybrene (Morgan, J. R., et al., Biotechnol. Prog., 10, 441-446, 1994), polyetheleneimines (Meunier-Durmort C., et al., Gene Therapy, 4, 808-814, 1997), cationic liposomes (Hodgson C. P. and Solaiman F., Nature Biotechnol., 14, 339-342, 1996), and synthetic polyamino acids (Curiel D. T., et al., Proc. Natl. Acad. Sci. USA., 88, 8850-8854, 1991).

However since both polybrenes and polyethylene imines are substances that are not present in the living body, their administration to the body will be problematic. With respect to cationic liposomes, it has been reported that their toxicity increases depending on the compositions prepared therefrom (Yagi K. et al., Biochem. Biophys. Res. Commun. 196, 1042-1048, 1993), and cautions will be needed. Furthermore, synthetic polyamino acids, particularly polylysine, tend to form precipitates with increasing concentrations, and thus they find difficulties in being used in the actual treatment of diseases. Especially when a drug solution containing particles that likely form precipitates in veins is administered, it can be the cause responsible for blockade of blood vessels, thrombus and the like. Even in the case of local administration, there is a problem such as the clogging of syringe needles; and another problem is that it is not possible to transfer into the target cells, a gene in an amount sufficient to. carry out treatment.

### Disclosure of the Invention

It is an object of this invention to provide a nucleic acid carrier for the efficient and safe transfer of a therapeutic gene into cells.

As a result of having pursued diligent investigations to solve the above-stated problems, the present inventors found that when a nucleic acid carrier comprising a polypeptide comprising diaminobutyric acid and/or a pharmaceutically acceptable salt thereof and a recombinant viral vector was used, the therapeutic gene could be transferred into cells efficiently and safely and thus, this invention was accomplished.

Specifically, this invention provides a nucleic acid carrier comprising a polypeptide comprising a diaminobutyric acid and/or a pharmaceutically acceptable salt thereof and a recombinant viral vector.

Also, the invention provides a nucleic acid carrier as described above wherein the polypeptide comprises a block copolymer of a polypeptide comprising diaminobutyric acid and/or a pharmaceutically acceptable salt thereof and polyethylene glycol.

Further, the invention provides a nucleic acid carrier as described above wherein the polypeptide comprises diaminobutyric acid and/or a pharmaceutically acceptable salt thereof, each having a residue number of from 20 to 280.

Also, the invention provides any nucleic acid carrier as described above wherein the recombinant viral vector

contains a therapeutic gene.

Further, the invention provides any nucleic acid carrier as described above wherein the recombinant viral vector is a HIV vector.

Additionally, the invention provides a nucleic acid carrier as described above wherein the HIV vector is a pseudo-type HIV vector with VSV-G.

Further, according to the invention, there is provided an agent for improving the gene transfer of a recombinant viral vector, said agent comprising as the effective agent, a polypeptide comprising a diaminobutyric acid and/or a pharmaceutically acceptable salt thereof.

## Brief Description of the Drawings

Fig. 1 is a representation showing an example of the synthetic pathway for the diaminobutyric acid and a pharmaceutically acceptable salt thereof according to this invention.

Fig. 2 is a schematic representation showing the construction of helper plasmid pCMV-R8.2 that constitutes a recombinant viral vector according to the invention.

Fig. 3 is a schematic representation showing the construction of plasmid pMD.G for providing VSV-G that constitutes a recombinant viral vector according to the invention.

Fig. 4 is schematic representation showing the construction of vector plasmid pHXCAGEGFP that constitutes a recombinant viral vector according to the invention.

Fig. 5 is a graph showing the results of the EGFP gene transfer by the GFP/HIV vector/PDBA complex that is a nucleic acid carrier of the invention.

Fig. 6 is a graph showing the results of the stability test for the GFP/HIV vector/PDBA complex that is a nucleic acid carrier of the invention.

## Best Mode for Carrying Out the Invention

The constitution and preferred embodiments of this invention will be described in detail hereafter. (Polypeptide Comprising Diaminobutyric Acid and/or a Pharmaceutically Acceptable Salt Thereof)

A polypeptide comprising diaminobutyric acid according to this invention has the structure represented by formula (1) wherein "n" represents a natural number and it may optionally contain an additional monomer unit.

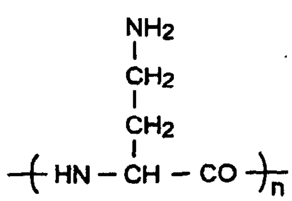

(1)

A polypeptide comprising a pharmaceutically acceptable salt of diaminobutyric acid diaminobutyric acid according to this invention has the structure represented by formula (2) wherein "n" represents a natural number and it may optionally contain an additional monomer unit.

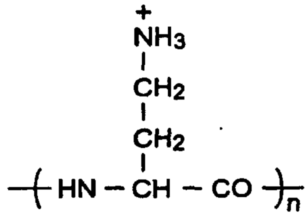

(2)

As used in this specification, the "polypeptide comprising diaminobutyric acid and/or a pharmaceutically acceptable salt thereof" may be such that it has a structure including the two structures described above in any proportion. Namely, embraced is that which exists in the form of diaminobutyric acid or a pharmaceutically acceptable salt thereof in any proportion.

In the diaminobutyric acid D-form and L-form can exist which are optical isomers. The polypeptide comprising diaminobutyric acid and/or a pharmaceutically acceptable salt thereof according to this invention includes D-form, L-form or a polypeptide that is a mixture of the foregoing in any proportion. In other words, it may be a polypeptide containing any of poly-L-diaminobutyric acid, poly-D-diaminobutyric acid, and poly-DL-diaminobutyric acid. As used in the specification, the "residue of a polypeptide comprising diaminobutyric acid and/or a pharmaceutically acceptable salt thereof" refers to a diaminobutyric acid (or a pharmaceutically acceptable salt thereof) moiety which is a monomer forming the polypeptide; and the number of residues refers to the number of the molecules (represented by "n" in the above formulae). The preferred number of residues of the polypeptide according to the invention comprising diaminobutyric acid and/or a pharmaceutically acceptable salt thereof is at least 10 residues. More preferably, the number of residues is at least 20 residues, and most preferably at least 25 residues. The preferred number of residues of the polypeptide according to the invention comprising diaminobutyric acid and/or a pharmaceutically acceptable salt is 280 residues or less. More preferably, the number of residues is 250 residues or less. When the number of residues is too large, the synthesis will be difficult and handling will also be inconvenient. On the other hand, when the number of residues is too small, performance as a nucleic acid carrier will be inadequate. Further, an optimal selection can be made as to the preferred number of residues depending on the recombinant viral vector to be used and the properties of components that can be used concurrently.

The pharmaceutically acceptable salt of diaminobutyric acid is not particularly limited insofar as it does not exhibit toxicity or it is a salt exhibiting toxicity at such a level that is tolerable. Preferably mentioned are, among others, inorganic acids such as hydrochloric acid, sulfuric acid, nitric acid, and phosphoric acid and organic acids such as acetic acid, propionic acid, citric acid, lactic acid, oxalic acid, succinic acid, tartaric acid, malonic acid, fumaric acid, and malic acid. Among these, acetate is particularly preferred.

Assuming that the polypeptide comprising diaminobutyric acid and/or a pharmaceutically acceptable salt thereof is polydiaminobutyric acid acetate, the number of residues described above can possibly be expressed in terms of molecular weight because the polydiaminobutyric acid acetate has a molecular weight of 160.

Another form of the polypeptide according to this invention comprising diaminobutyric acid and/or a pharmaceutically acceptable salt thereof (which will be referred to as "the polypeptide according to this invention" hereafter) includes one having a block copolymer structure where polyethylene glycol is further linked to the polypeptide comprising diaminobutyric acid described above and having formula (3) wherein "n" and "m" represent a natural number, respectively.

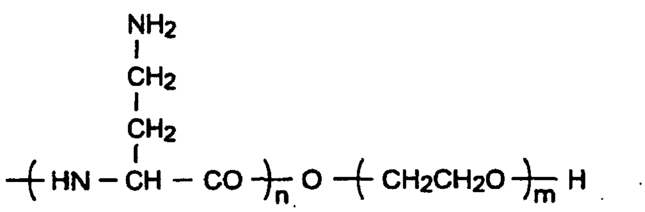

(3)

Further, since the polypeptide has a carboxylic acid moiety, a copolymer represented by formula (4) wherein "m," "n," and "n' " represent a natural number, respectively is possibly one of those having the block copolymer structure.

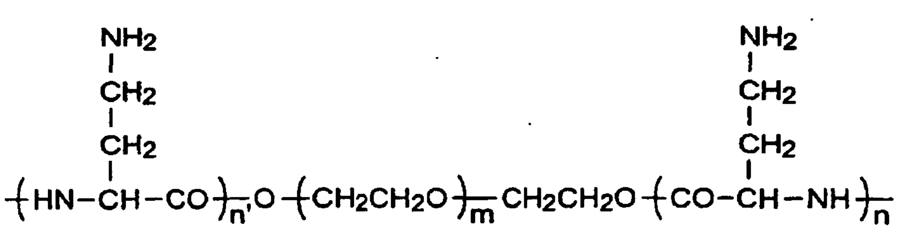

(4)

As used herein, the molecular weight of ethylene glycol (or number "m") is not particularly limited, but it is normally on the order of from 200 to 25,000 for the reasons that will be described later.

In an embodiment of this invention a tissue-specific ligand can be linked to the polypeptide according to this invention. The nucleic acid carrier of this invention containing such a tissue-specific ligand is effectively or in a tissue-specific manner incorporated into a particular tissue to which the ligand is specific. For example, when the liver is targeted, a sugar (glactose, lactose, acyloglycoprotein, oligoglactose, hyaluronic acid, etc.) is linked to the polypeptide according to this invention with the result of increased affinity to the liver, the more effective delivery of the nucleic acid carrier of this invention to the liver will be possible.

In order to reduce cytotoxicity, to improve the residence ability in blood, and to further improve solubility in solvent, it is preferred that a block copolymer of diaminobutyric acid and polyethylene glycol (PEG) as represented by formula (3) or formula (4) be employed as the polypeptide according to this invention. In this instance, PEG having a molecular weight of 200 or more is preferably used. More preferably, PEG having a molecular weight of 1,000 or more is used. The molecular weight of PEG is preferably 25,000 or less, more preferably 10,000 or less, and most preferably 5,000 or less. If the molecular weight of PEG is 25,000 or more, affinity to the cell surface decreases, thus resulting in lowered efficiency of nucleic acid (gene) transfer; if the molecular weight is 200 or less, the intended effect by polyethylene glycol will be small, which is undesirable.

(Synthetic Method for Polypeptides According to This Invention)

There is no particular limitation to the method for synthesizing the polypeptides according to this invention that are characterized by having the structures described above. Preferably, usable are organic chemical reactions including a variety of synthetic methods for polypeptides that are ordinarily known (New Experimental Chemistry Monograph No. 19 Polymer Chemistry I, published in 1980, Maruzen Co. Ltd.).

More specifically, it is preferred in this invention that monomer components be polymerized through a suitable reaction. In this case, the monomers to be used may preferably employ diaminobutyric acid, diaminobutyric acid where a protective group has been introduced into the γ-amino group, or diaminobutyric acid having activated amino groups and/or carboxylic acid groups for peptide group formation. Especially, in this invention it is preferred that the γ-amino group of diaminobutyric acid is protected and the diaminobutyric acid is then converted to an acid anhydride thereof in order to render the peptide bond formation easy (New Experimental Chemistry Monograph No. 19, Polymer Chemistry I, published in 1980, Maruzen Co. Ltd.).

In order to polymerize such monomers, it is possible to use various kinds of initiators in appropriate quantities. Concretely, various amine and alcohol compounds are usable. The amine compounds include alkylamines; particularly, butylamine is preferable. When polyethylene glycols are used as alcohols, the polypeptides according to this invention (which comprises a block copolymer of diaminobutyric acid and polyethylene glycol) may be obtained that have polyethylene glycol moieties attached.

For the synthesis of polypeptides according to this invention, the particularly preferred synthetic pathway is illustrated in Fig. 1. Specifically, it is preferred that the γ-amino group be protected with a suitable protecting group and the amino acid portion be converted, using phosgene, into an acid anhydride which is to be used as a monomer in polycondensation ((10) in Fig. 1). Employing this monomer, the polycondensation reaction allows suitable initiators to be used; and it becomes possible to introduce a preferable number of monomers. Although the initiator is not particularly limited, various amine compounds are preferable, and the use of butylamine is particularly preferable. When suitable ethylene glycols are used as initiators, it will be possible to obtain block copolymers having ethylene glycol moieties which are polypeptides having preferable numbers of monomers.

In practice, the methods as described in Helv. Chim. Acta, 43, 270 (1960) or in New Experimental Chemistry Monograph No. 19, Polymer Chemistry I, published in 1980, Maruzen Co. Ltd. may be followed, or modified to be carried out.

(Detection of The Polypeptides According to This Invention)

The structures of the polypeptides according to this invention have the characteristics described previously. Therefore, it will be possible to detect the polypeptides according to this invention comprising diaminobutyric acid and/or a pharmaceutically acceptable salt thereof based on such structural characteristics. Even if the polypeptides according to this invention themselves, or the nucleic acid carriers or pharmaceutical compositions for gene therapy using them are used in different forms, the detection of polypeptides according to this invention will be likewise possible when suitable pretreatment is performed. One skilled in the art can readily select the necessary treatment.

The method of detection is not particularly limited; and a variety of ordinarily known polypeptide analysis methods can be used (Young H. C. J. Controlled Release 54, 39-48, 1998). Concretely usable are the qualitative and quantitative analysis of diaminobutryic acid following the amino acid assay method in peptides, the determination of the number of residues based on molecular weight measurement using various kinds of liquid chromatography, various spectral analyses for detecting the presence of polyethylene glycol moieties (infrared absorption spectroscopy and

nuclear resonance absorption spectroscopy), mass spectroscopy, and chemical qualitative analysis methods.

(Recombinant Viral Vectors)

The recombinant viral vectors according to this invention may be prepared from DNA viruses or RNA viruses. The virus species from which the recombinant viral vector is derived is not particularly limited and it may be any viral vector of an MoMLV vector, a herpes virus vector, an Adenovirus vector, an AAV vector, a HIV vector, a Seidai virus vector and the like. Further, viruses having a host spectrum other than human are usable as the viral vector insofar as they will be efficacious.

For the recombinant viral vector according to this invention, a HIV vector is used most preferably. Because the HIV vector incorporates a transferred nucleic acid into its chromosome, it allows the nucleic acid (i.e., a therapeutic gene) to be expressed over a prolonged period of time. The HIV vector also allows for the selective gene transfer into CD4 positive T cells which are cell surface molecules. Furthermore, the HIV vector allows for the chromosome incorporation even at the resting phase when the cells are not dividing; therefore, it will be possible to efficiently transfer a therapeutic gene into any of bone marrow stem cells, hematopoietic stem cells, nerve cells and muscle cells at their respective resting phases.

One or more proteins among the constituent protein group for the viral vector are substituted by the constituent proteins of a different species of virus, or alternatively a part of the nucleic acid sequence constituting genetic information is substituted by the nucleic acid sequence of a different species of virus to form a viral vector of the pseudo-type, which can be used as a recombinant viral vector according to this invention. For example, there is mentioned a pseudo-type viral vector wherein the Env protein (an envelop protein of HIV) is substituted by the VSV-G protein (an envelop protein of vesicular stomatitis virus or VSV) (Naldini L., et al., Science 272, 263-267, 1996).

The recombinant HIV vectors used in this invention and the methods of their production will be described in detail hereunder.

Specifically, a helper plasmid (pCMV-R9) is constructed according to genetic manipulations known in the art: a group of genes are prepared that contain the gag, pol, and env genes of HIV and that lack packaging signals and the promoter of cytomegalovirus (CMV) is arranged at their upstream to form a gene construct which is then inserted into an expression vector containing the ampicilin-resistant gene and the replication origin of SV40. pCMV-R8.2 that does not express the Env protein is further constructed according to genetic manipulations known in the art by deleting a portion of the env gene of pCMV-R9. The aforementioned gene group can be constructed from the genome sequence of a mammalian immunodeficiency virus, including HIV-1, HIV-2, simian immunodeficiency virus, and feline immunodeficiency virus. A recombinant HIV virus of the pseudo-type the envelope protein of which is VSV-G can be prepared by co-transfecting cells with the helper plasmid, the pMD.G plasmid that expresses VSV-G protein, and the vector plasmid described below.

It is possible to reduce the probability of occurrence of RCR (replication competent retrovirus) by eliminating packaging signals from the helper plasmid. Substitution of the Env protein with the VSV-G protein will further allow gene transfer into the cells that do not express CD4 (Naldini L. et al., Science 272, 263-267, 1996).

If one or more of the vif, vpr, vpu, tat, rev, nef genes (other than the gag, pol, and env genes essential for the constitution of HIV), or their corresponding genes are arbitrarily deleted, the safety can be increased. The promoters used to express HIV genes are not limited to CMV and , for example, there may be used promoters such as CAG, RSV, TK, SV40, SR-$\alpha$, $\beta$-actin, and EF1-$\alpha$.

Addition of an enhancer sequence that enhances a promoter through a viral enhancer protein such as herpes virus or hepatitis B virus or through a cellular transcriptional activation protein such as NF $\kappa$-B or SP-1 is possible. A recombinant HIV vector can be constructed by co-transfection with a plasmid encoding any of these proteins. The cells for preparing a recombinant vector can employ any cell line insofar as they are cells capable of producing HIV proteins. Preferably, there are mentioned mammalian cell lines such as CV-1, HeLa, Raji, RD, SW480, CHO, COS, 293 cells, and 293T cells obtained from the transformation of SV40 large T antigen. Especially, COS and 293T cells are preferable and the 293T cells are most preferable.

The fragment required to have the therapeutic gene expressed is packaged into HIV virions, subjected to reverse transcription by target cells and incorporated into the chromosome. It is represented in the 5'-end to 3'-end direction by the following: HIV-LTR (or Long-Terminal-repeats consisting of U3, R, and U5 regions), packaging signals, any promoter to express a therapeutic gene, the therapeutic gene and HIV-LTR. A vector plasmid is constructed according to gene manipulations known in the art: the gene construct where the foregoing are arranged is, for example, inserted into an expression vector containing the ampicilin-resistant gene and the replication origin of SV40. Here, the vector plasmid can be constructed from the genome sequences of a mammalian immunodeficiency virus, including HIV-1, HIV-2, simian immunodeficiency virus (SIV), and feline immunodeficiency virus (FIV).

A polyadenylation signaling sequence may be appended to the therapeutic gene if necessary. There is no particular limitation to the promoter to be arbitrarily selected that allows the therapeutic gene to be expressed. Specifically, there may be used promoters such as CAG, CMV, RSV, TK, SV40, SR-α, MBP, β-actin, and EF1-α. For the therapeutic gene and LTR at the 5'-end of the promoter for expressing the therapeutic gene, there may be used a LTR of the chimera type wherein the U3 region of LTR has been replaced by a prompter such as CAG, CMV, RSV, TK, SV40, SR-α, MBP, β-actin, or EF1-α.

The plasmids thus constructed are subjected to co-transfection into COS cells, for example, by the known technique such as the calcium phosphate method, the lipofection methods using various cationic lipids or the electroporation method.

To efficiently construct a recombinant HIV vector, the infected cells are cultured at an optimal temperature for the host cells used for 24 to 72 hours; culturing at least 48 hours is preferable.

The recombinant HIV vector released into the culture supernatant can be prepared into a recombinant HIV vector solution by centrifuging the culture supernatant at 2,000 rpm for 10 minutes, or alternatively by removing contaminants derived from the host cells through a 0.45 μm-filter. Further, the contaminants may be removed by the density gradient centrifugation, the PEG (polyethylene glycol) precipitation method, or column techniques using columns such as cellulose sulfate column and affinity column.

(Therapeutic Genes)

The therapeutic gene to be inserted into the recombinant viral vector according to this invention and other genes are comprised of various nucleic acids and/or nucleotide derivatives; and there are no particular limitations to their kinds, molecular weights, shapes, and sequences.

Specifically, for the shape of nucleic acid, a single-stranded gene, a double-stranded gene, a triple-stranded forming gene, DNA, RNA, a DNA/RNA chimera-type gene, a phosphothioate-type gene, a straight-chain gene, a circular gene, and the like may be used without any restriction. The sequence of gene to be encoded within the recombinant viral vector can employ, in addition to the therapeutic gene, any sequence among a promoter or enhancer for the transcription of the therapeutic gene, a poly-A signal, a marker gene for labeling and/or selecting the cell into which the gene has been introduced, a virus-derived gene sequence for the efficient insertion of gene into cellular genomic DNA sequences, and a signal sequence for extracellularly secreting the substance that acts as drug and/or for having the substance remained at localized sites within a cell.

For the therapeutic genes, genes corresponding to disorders, namely genes that act against the disorders in an antagonistic manner or genes that supplement those lacking in the disorders may be used. Specifically, there are mentioned genes encoding a pharmacologically active compound selected from the group consisting of a cytokine, a growth factor, antibody, an antibody fragment, a receptors against cytokine or growth factor, a proteins having proliferation preventive or cell proliferation inhibitory action, enzyme, a pulse-related suppressor, a thrombus-inducing substance, a coagulation suppressor, a protein having fibrin-dissolving action, a viral coat protein, a bacterial antigen and a host animal antigen, a tumor antigen, a protein effective for blood circulation, a peptide hormone, ribozyme, and a ribonucleic acid such as antisense RNA.

Representatives of the genes against specific disorders include: SOD, anti-inflammatory cytokines, and a gene encoding the peptide that act on a cell-adhesion factor in an antagonistic manner for an inflammatory disorder; a gene encoding a normal enzyme for an enzyme-deficient disorder; a gene encoding a normal receptor for a receptor-deficient disorder; a thymidine kinase that kills virus-infected cells, a gene encoding a toxin such as diphtheria toxin, a gene encoding antisense, triplehelix, ribozyme, a decoy, or a transdominant mutant each of which inhibits the replication of virus for a virus infection; a thymidine kinase that kills cancer cells, a gene encoding a toxin such as diphtheria toxin, a gene encoding antisense, triplehelix, or ribozyme each of which inactivates a cancer gene, a cancer-suppressing gene such as p53 that normalize the cancer cells, a gene encoding antisense, triplehelix, or ribozyme each of which inactivates a gene that is involved in the multi-drug resistance against an anti-cancer agent for cancer; and a gene encoding a LDL receptor for familial hypercholesterolemia.

As for the expression cassettes to be used for the therapeutic gene, any cassettes without any particular limitations may be used insofar as they can cause genes to express in the target cells. One skilled in the art can readily select such expression cassettes. Preferably, they are expression cassettes capable of gene expression in the cells derived from an animal, more preferably, expression cassettes capable of gene expression in the cells derived from a mammal, and most preferably expression cassettes capable of gene expression in the cells derived from human. The gene promoters that can be used as expression cassettes include: for example, virus-derived promoters from Adenovirus, cytomegalovirus, human immunodeficiency virus, simian virus 40, Rous sarcoma virus, herpes simplex virus, murine leukemia virus, sinbis virus, Sendai virus, hepatitis type A virus, hepatitis type B virus, hepatitis type C virus, papilloma virus, human T cell leukemia virus, influenza virus, Japanese encephalitis virus, JC virus, parbovirus B19, poliovirus, and the like; mammal-derived promoters such as albumin and a heat shock protein; and chimera type promoters such as a CAG promoter.

(Nucleic Acid Carriers)

The nucleic acid carrier of this invention is characterized in that it contains the aforementioned polypeptide comprising diaminobutyric acid and/or a pharmaceutically acceptable salt and the aforementioned recombinant viral vector. When the polypeptide and the therapeutic gene are combined at a predetermined ratio, they readily form a complex, leading to the nucleic acid carrier of this invention. According to a preferred embodiment of this invention, a recombinant viral vector of the invention already contains a suitable therapeutic gene as described above. When this recombinant viral vector and a polypeptide according to the invention are combined, a nucleic acid carrier that can be used in gene therapy and that is also a therapeutic composition for gene therapy results. The therapeutic composition for gene therapy may be prepared by mixing the recombinant viral vector containing a therapeutic gene and the polypeptide comprising diaminobutyric acid and/or a pharmaceutically acceptable salt. The scope of this invention encompasses the therapeutic composition for gene therapy thus produced.
More specifically, after the recombinant viral vector containing a therapeutic gene and the polypeptide comprising diaminobutyric acid and/or a pharmaceutically acceptable salt are separately dissolved in appropriate solvents such as water, physiological saline water, or an isotonicated buffer, they are mixed, thus enabling the preparation of a therapeutic composition for gene therapy.
Here, the mixing ratio of the recombinant viral vector containing a therapeutic gene for use to the polypeptide according to this invention is not particularly limited. In practice, the polypeptide according to this invention is used at a concentration of from 5 μg/ml to 20 μg/ml, preferably from 5 μg/ml to 10 μg/ml relative to 1 ml of the recombinant viral solution at from $1x10^2$ to $1x10^{13}$ IU/ml, preferably $1x10^3$ to $1x10^{12}$ IU/ml, more preferably $1x10^4$ to $1x10^{11}$ IU/ml.
As used herein, "IU (infectious unit)" refers to the gene transfer efficiency (or titer) of a recombinant viral vector and represents the number of cells into which one milliliter of the viral vector solution can transfer gene. After arbitrary cells are subjected to gene transfer by the recombinant viral vector containing the neomycin drug-resistant gene, the cells are grown in a culture containing the drug. The gene transfer efficiency is then obtained by counting the number of drug-resistant cells that have grown.
The nucleic acid carrier of this invention forms a complex even one minute after mixing the polypeptide and the recombinant viral vector, and this complex exists in the mixed solution stably after a lapse of one hour upon its formation.
The nucleic acid carrier of this invention does not form precipitates after mixing and complex formation. Therefore, when the nucleic acid carrier of this invention is directly administered to the blood vessels, there is no danger of thrombus formation, thus allowing for efficient and precise administration of the therapeutic gene.
The nucleic acid carrier of this invention may further contain additives as required. For example, such additives may be added to the peptide of this invention as required for the purpose of the promotion of cell-adhesion, stabilization of the recombinant viral vector, accelerated transfer of the therapeutic gene into the cell or the nucleus, or the controlled release of the recombinant viral vector.
These additives are not particularly limited insofar as they accomplish the above-mentioned objects, and there may be mentioned among others glycine, sodium glutamate, chondroitin sulfate, gelatin, albumin, cytokine, a HMG-1 and HMG-2 mixture, chloroquine, VSV-G (an envelop protein of vesicular stomatitis virus), and penton (Adenovirus structural protein).
The nucleic acid carrier of this invention allows the therapeutic gene to be transferred into the target cells and to be expressed efficiently over about three-fold as compared to when the recombinant viral vector alone is used to transfer the therapeutic gene.

(Methods of Use of the Nucleic Acid Carrier of This Invention)

The nucleic acid carrier of this invention can be used in the gene therapy through autologous implantation (ex vivo gene therapy) where the target cells are first removed outside the body from the patient and the cells are then returned to the body of the patient after the therapeutic gene has been transferred into the cells. The therapeutic gene can also be used in the gene therapy where the therapeutic gene is directly administered to the patient (in vivo gene therapy).
Gene therapy is largely classified into "Augmentation Gene Therapy" in which aberrant (causative) genes are left intact and new (normal) genes are augmented and "Replacement Therapy" in which aberrant genes are replaced with normal genes. The nucleic acid carriers of this invention can be used in both types of therapy.
Methods for administering the nucleic carrier of this invention to the body are not particularly limited. They may preferably be carried out by, for example, parental administration such as administration through injection.
The dosages for the nucleic acid carrier of this invention differ depending on the intended methods, the intended objects, etc., and one skilled in the art can readily determine an optimal dosage for the subject to be treated. Specifically, where administration by injection is used, preferably administration is done in a daily dose of from about

0.1 µg/kg to about 1,000 mg/kg, and more preferably in a daily dose of from about 1 µg/kg to about 100 mg/kg.

## EXAMPLES

This invention will be described more concretely by referring to the preparations, the actual examples and the test examples; however, the invention is not to be limited by these examples.

The synthesis of the polypeptides comprising diaminobutyric acid and/or a pharmaceutically acceptable salt thereof used in the preparation examples and the examples below was carried out according to the method of K. Vogeler et al. (Helv. Chim. Acta, 43, 270 (1960)). The synthetic pathway is shown in Fig. 1.

In the present specification, polydiaminobutyric acid (poly(2,4-diaminobutyric acid)), including polydiaminobutyric acid acetate are abbreviated as "pDBA." The pDBA is defined to encompass those based on all possible optical isomers. Likewise block copolymers with polyethylene glycol are abbreviated as "pDBApeg" (or pDBA-PEG).

(Preparation 1) <u>Synthesis of polypeptide comprising diaminobutyric acid and/or a pharmaceutically acceptable salt</u>

(I) Synthesis of N-γ-carbobenzoxy-DL-diaminobutyric acid NCA (4N-carbobenzoxy-DL-2,4-diaminobutyric acid N-carboxyanhydride (10) as a monomer

(I-1) Synthesis of N- γ-carbobenzoxy-DL-diaminobutyric acid (4N-carbobenzoxy-DL-2,4-diaminobutyric Acid) (8)

DL-2,4-diamino-n-butyric acid dihydrochloride (1)(15 g, Sigma-Aldrich Corporation) was dissolved in 75 ml of water. To this was added basic cupper carbonate (2) (11.7 g), and it was allowed to stand. Then it was boiled at reflux, and it was filtered. Sodium bicarbonate (16.6 g) and carbobenzoxyl chloride (4) (17.8 ml, Wako Pure Chemical Industries, Ltd.) were added to the filtrate and stirred to obtain product as a precipitate. The resulting product was filtered and washed with acetone and diethyl ether. Then drying produced 15 g of N-γ-carbobenzoxy-DL-diaminobutyric acid cupper complex (4N-carbobenzoxy-DL-2,4-diaminobutyric acid copper complex (hereafter referred to as "Dba(Z) -Cu") (5).

The resulting complex (5), 11 g, was added to a mixed solution of 35% HCl (9.3 ml), water (22 ml) and methanol (11 ml) and stirred in the presence of hydrogen sulfide ($H_2S$) gas. After allowing the solution to stand at room temperature, excessive hydrogen sulfide was removed and then insoluble materials were removed by filtration. The filtrate was subjected to reduced pressure and was cooled after addition of water and methanol under. After further addition of methanol, the pH of the solution was adjusted to 7 by addition of diethylamine (7). The precipitated crystals were separated by filtration and washed on a filter funnel with diethyl ether. After drying, 4g of product was obtained as a white crystal. Further, the filtrate was concentrated to precipitate crystals. The crystals were separated by filtration and washed on the filter funnel with diethyl ether. After drying, 1.5 g of product was obtained as a white crystal. These crystals were combined to yield a total of 5.5 g of N-γ-carbobenzoxy-DL-diaminobutyric acid (8) (31% yield calculated from the starting amino acid).

(I-2) Synthesis of N- γ-carbobenzoxy-DL-diaminobutyric acid NCA (4N-carbobenzoxy-DL-2,4-diaminobutyric acid N-carboxy-anhydride) (10)

The compound obtained above (8), 5 g, was dissolved in tetrahedrofuran (THF, 200 ml). To this was added 4.5 g of triphosgene (9) (bis (tricholoromethyl) carbonate available from Sigma-Aldrich Corporation) dissolved in 40 ml of THF, and it was stirred at 40 °C for 60 minutes. After removal of solvent under reduced pressure, hexane was added to the resulting crude product for dissolution. After cooling and further removal of hexane thoroughly under reduced pressure, ethyl acetate was added to the resulting product for dissolution, and the insoluble materials were removed by filtration. When the resulting filtrate was cooled after addition of hexane, product was precipitated as a white crystal. The precipitated crystals were separated by filtration and dried under reduced pressure. The filtrate was concentrated under reduced pressure, and then it was similarly treated to produce product as a crystal. The obtained crystals were recrystallized from diethyl ether to produce 2.7 g (50% yield) of the purified product (10).

(II) Polymerization

Polypeptides comprising various numbers of residues were obtained by subjecting the obtained N-γ-carbobenzoxy-DL-diaminobutyric acid NCA (10) to condensation polymerization using initiators in different proportions and thereafter, by deprotecting the protecting groups for the amino groups.

As used herein, the number of residues according to this invention refers to that calculated following the equation below (Arieh Yaron et al., Biochim. Biophys. Acta, 69, 397-399, 1963): multiplication by 0.9 at the end of

equation has such reason that the molecular weight decreases about 10% under the reaction conditions for removing protective groups. Further, the molecular weights in this invention refer to those expressed by the following equation:

The number of residues=the degree of polymerization=[the quantity of N-γ-carbobenzoxy-DL-diaminobutyric acid NCA (10) (mole number)/the quantity of initiator (mole number)] x yield (%)/100 x 0.9

Molecular weight=n (the degree of polymerization) x quantity of residue (the quantity of residue for DBA acetate as acetate=160)

The synthesis of poly-DL-diaminobutyric acid (poly(DL-2,4-diaminobutyric acid)) and its acetate shown in Synthetic Example 3 (the number of residues=49) in Tables 1 and 2 is described in detail below. Employing the other conditions shown in Table 1, pDBAs in Synthetic Example 1 (the number of residues=12), Synthetic Example 2 (the number of residues=26), Synthetic Example 4 (the number of residues=62), Synthetic Example 5 (the number of residues=170), Synthetic Example 6 (the number of residues=278), and Synthetic Example 7 (the number of residues=348) were obtained similarly. Synthetic conditions for polydiaminobutyric acid-PEG (15) obtained by linking polyethylene glycol (14) (PEG, molecular weight=1,000) to polydiaminobutyric acid and its acetate of Synthetic Example 3 at their ends are shown as Synthetic Example 8 in Tables 1 and 2.

(II-1) Synthesis of poly-N-γ-carbobenzoxy-DL-diaminobutyric acid (poly(4-N-carbobenzoxy-DL-2,4-diaminobutyric acid) (11)

N-γ-carbobenzoxy-DL-diaminobutyric acid NCA (10) (1 g, 3.6 mmol) was dissolved in 19 ml of acetonitrile.

Butylamine (4.38 mg, 0.06 mmol) was added as an initiator to the solution, which was then allowed to stand at 30 °C for 307 hours. The resulting polymer was filtered and washed with acetonitrile. After extraction with diethyl ether using a Soxlet extractor, it was dried under reduced pressure to produce 0.77 g of poly-N-γ-carbobenzoxy-DL-diaminobutyric acid (11) (the degree of polymerization=91%).

(II-2) Synthesis of poly-DL-diaminobutyric acid (poly(DL-2,4-diaminobutyric acid) acetate (13)

Poly-N-γ-carbobenzoxy-DL-diaminobutyric acid (11), 0.5 g, was dissolved in 2 ml of trifluoroacetic acid, to which 25% hydrogen bromide acetate solution (12) was added and mixed with shaking. After allowing the solution to stand, diethyl ether was added thereto and the supernatant ether phase was removed by decantation. The same manipulation was carried out with diisopropyl ether and the supernatant ether phase was removed by decantation. The resulting precipitates were sufficiently brought to dryness under reduced pressure. Sodium acetate and water were added to the obtained solids to produce a mixed solution. The mixed solution was dialyzed with running water using a dialysis tube that would remove materials with a molecular weight of 1,000 or less. Then, centrifugation was carried out at 20,000G for 1 hour to remove precipitates. The resulting solution was lyophilized to produce 0.34 g of poly-DL-diaminobutryic acid acetate (13)(yield 91%).

【table 1】

Synthetic Conditions for Poly(4-N-carbobenzoxy-DL-2,4-diaminobutyric acid) by Polycondensation of 4-N-Carbobenzoxy-DL-2,4-diaminobutyric Acid NCA

| Synthetic Example | NCA g(mmol) | Initiator a) mg(mmol) | A/I b) | Solvent c) (ml) | Monomer (mol/l) | Temperature Time | Yield |
|---|---|---|---|---|---|---|---|
| 1 | DL-(Z)diaminobutyric acid 0.53g (1.9) | BA 7.7(0.105) | 18 | ACN;15 | 0.127 | 30℃ 312hr | 0.35g (76%) |
| 2 | DL-(Z)diaminobutyric acid 1g (3.6) | BA 8.21(0.11) | 32 | ACN;20 | 0.18 | 30℃ 307r | 0.754g (90%) |
| 3 | DL-(Z)diaminobutyric acid 1g (3.6) | BA 4.38(0.06) | 60 | ACN;19 | 0.19 | 30℃ 307r | 0.773g (91%) |
| 4 | DL-(Z)diaminobutyric acid 0.705g (2.53) | BA 2.22(0.03) | 83 | ACN;20 | 0.127 | 30℃ 312hr | 0.491g (83%) |
| 5 | DL-(Z)diaminobutyric acid 0.705g (2.53) | BA 0.88(0.012) | 208 | ACN;20 | 0.127 | 30℃ 312hr | 0.539g (91%) |
| 6 | DL-(Z)diaminobutyric acid 1.0g (3.6) | BA 0.77(0.011) | 340 | ACN;19 | 0.19 | 30℃ 360hr | 0.764g (91%) |
| 7 | DL-(Z)diaminobutyric acid 1.0g (3.6) | BA 0.60(0.008) | 440 | ACN;19 | 0.19 | 30℃ 360hr | 0.739g (88%) |
| 8 | DL-(Z)diaminobutyric acid 0.78g (2.8) | PEG1000 60(0.06) | 46 | ACN;175 | 0.19 | 30℃ 307hr | 0.559g (76%) |

a) BA ; butylamine

PEG1000 ; polyethylene glycol MW 1000

b) N-Carboxy Anhydride (NCA) / Initiator mol ratio

c) ACN ; Acetonitrile

【table 2】

Synthetic Conditions for Poly(DL-2,4-diaminobutyric acid) Acetate by Deprotection of Poly(4-N-carbobenzoxy-DL-2,4-diaminobutyric Acid)

| Synthetic Example | Component a) (g) | TFA-HBrHAc (ml) | Temperature Time | NaOAc(g) $H_2O$(m) | Dialysis Time | Centrifugation Temperature | Yield | Number of Residue | Molecular Weight |
|---|---|---|---|---|---|---|---|---|---|
| 1 | Poly(Dba(Z)) 0.306 | 1.3-1.3 | 24℃ 1.8hr | 0.31g 20ml | 500cut 17hr | 30,000rpm 4℃, 1hr | 0.115g (46%) | 12 | 1,900 |
| 2 | Poly(Dba(Z)) 0.509 | 2-2 | 22℃ 2hr | 0.5g 25ml | 1000cut 17hr | 30,000rpm 4℃, 1hr | 0.267g (77%) | 26 | 4,200 |
| 3 | Poly(Dba(Z)) 0.506 | 2-2 | 22℃ 2hr | 0.5g 25ml | 1000cut 17hr | 30,000rpm 4℃, 1hr | 0.339g (98%) | 49 | 7,800 |
| 4 | Poly(Dba(Z)) 0.352 | 1.5-1.5 | 24℃ 2hr | 0.35g 25ml | 1000cut 17hr | 30,000rpm 4℃, 1hr | 0.139g (58%) | 62 | 9,900 |
| 5 | Poly(Dba(Z)) 0.379 | 1.5-1.5 | 24℃ 1.8hr | 0.35g 25ml | 1000cut 17hr | 30,000rpm 4℃, 1hr | 0.195g (75%) | 170 | 27,200 |
| 6 | Poly(Dba(Z)) 0.379 | 1.5-1.5 | 24℃ 1.8hr | 0.35g 25ml | 1000cut 17hr | 30,000rpm 4℃, 1hr | 0.210g (81%) | 278 | 44,500 |
| 7 | Poly(Dba(Z)) 0.379 | 1.5-1.5 | 24℃ 1.8hr | 0.35g 25ml | 1000cut 17hr | 30,000rpm 4℃, 1hr | 0.207g (80%) | 348 | 55,700 |
| 8 | Poly(Dba(Z)) -PEG 0.416 | 2-2 | 22℃ 2hr | 0.5g 25ml | 1000cut 17hr | 30,000rpm 4℃, 1hr | 0.254g (78%) | 31 | 6,000 |

a)

Poly(Dba(Z)) ; Poly(4-N-Carbobenzoxy-DL-2,4-diaminobutyric acid)

Poly(Dba(Z))-PEG ; Poly(4-N-Carbobenzoxy-DL-2,4-diaminobutyric acid)-PEG100'

(Preparation 2) Preparation of GFP/HIV Vector

A gene construct containing the gag and pol genes of HIV as well as the promoter of cytomegalovirus arranged at the upstream of the group of genes lacking packaging signals was inserted into an expression vector containing the ampicilin-resistant gene and the replication origin of SV40 to form pCMV-R8.2 (Naldini L., et al., Proc. Natl. Acad. Sci. USA., 93, 11382-11388, 1996: Fig. 2). A gene construct containing the promoter of cytomegalovirus arranged at the upstream of the gene encoding VSV-G which was the envelop protein of vesicular stomatitis virus was incorporated into an expression vector pXF3 to form plasmid pMD.G (Naldini L., et al., Proc. Natl. Acad. Sci. USA., 93, 11382-11388, 1996: Fig. 3). A gene construct where HIV-LTR, the CAG promoter, enhanced green fluorescence protein (EGFP) gene and HIV-LTR were arranged from the 5'-end to 3'-end direction was inserted into an expression vector containing the ampicillin-resistant gene the replication origin of SV40 to form a vector plasmid pHXCAGEGFP (Fig. 4). pCMV-R8.2, pMD.G and pHXCAGEGFP were subjected to co-transfection into COS cells by the calcium phosphate method. After growing for 2 days, the resultant culture supernatant was centrifuged at 2,000 rpm for 10 minutes and the precipitates were removed to produce a GFP/HIV vector solution.

(EXAMPLE 1) Preparation of GFP/HIV Vector/PDBA Complexes

GFP/HIV vector prepared in Preparation 2 and PDBA solutions prepared at twice the desired concentrations were mixed in equal amounts just. prior to administration to prepare GFP/HIV vector /PDBA complex solutions that are nucleic acid carriers of this invention. Preparation was made such that the final concentrations of PDBA 0, 0.3, 1.0, 3.0, 5.0, 10.0, 15.0, 20.0, and 25.0 μg/ml, respectively. DMEM medium (Sigma-Aldrich Corporation) was used as solvent. PDBA having a molecular weight of 7,800 obtained in Synthetic Example 3 in Table 2 was used. Even when the complex was prepared at the final PDBA concentration of 25 μg/ml, no precipitates were formed in solution.

(TEST EXAMPLE 1) Gene transfer into HeLa cells by GFP/HIV vector/pDBA complex

HeLa cells were inoculated onto a 12-well multi-well plate (Coaster Inc.) at $1x10^5$ cells/well one day prior to testing. Administration of the GFP/HIV vector/PDBA complex solution was carried out in the presence of 10% fetal bovine serum (Sanko Junyaku Co. Ltd.), and incubation was carried out at 37 °C for 4 hours. Medium was exchanged with a fresh culture medium and incubation continued for 48 hours.

(TEST EXAMPLE 2) Measurement of gene transfer efficiency with a flow cytometer

The gene-transferred HeLa cells from Test Example 1 were treated with trypsin and recovered from the plate. The recovered cell suspension was fixed with 1% paraformaldehyde and was measured using a flow cytometer (FACS Calibur available from Becton Dickinson). Fig. 5 shows the proportions of the cells into which the marker gene (EGFP) was transferred among 10,000 cells. It was confirmed that genes had been transferred into about 30% of cells in the case of the recombinant HIV vector (PDBA 0 μg/ml) alone as control. In the group of the complexes of PDBA with GFP/HIV vector, gene transfer efficiency increased in a concentration-dependent manner relative to PDBA and about three-fold gene transfer efficiency was observed at final concentrations of over 10.0 μg/ml as compared to the control. In Fig. 5 the unit of ordinate is percentage and the graph represents gene transfer efficiency in terms of percentage when the control is taken as 100%.

(TEST EXAMPLE 3) The stability evaluation of GFP/HIV vector/PDBA complexes

(Preparation of GFP/HIV vector/PDBA complexes)

GFP/HIV vector prepared in Preparation 2 and a PDBA solution prepared at 20.0 μg/ml were mixed in equal amounts 1, 3, 10, 15, 30, or 60 minutes prior to administration to prepare GFP/HIV vector /PDBA complex solutions (the final concentration of PDBA: 10.0 μ g/ml).

DMEM medium (Sigma-Aldrich Corporation) was used as solvent. PDBA having a molecular weight of 7,800 obtained in Synthetic Example 3 in Table 2 was used. After allowing each of the GFP/HIV vector/PDBA complex solutions to stand at room temperature, it was administered to HeLa cells. Forty eight hours later cells were fixed and gene transfer efficiency was measured with a flow cytometer (FACS Calibur available from Becton Dickinson). Fig. 6 shows the proportions of the cells into which the EGFP gene was transferred among 10,000 cells. From Fig. 6 it was confirmed that PDBA and the recombinant viral vector formed a stable complex immediately after mixing and the gene transfer efficiency experienced no change even after allowing standing for one hour at room temperature. In Fig. 6 similarly to Fig. 5, the unit of ordinate is percentage and the graph represents gene transfer efficiency in terms of

percentage at each concentration when the control is taken as 100%.

### Industrial Applicability

The nucleic acid carrier of this invention is such that a polypeptide comprising diaminobutyric acid and/or a recombinant viral vector forms a complex and it allows a therapeutic gene to be transferred into cells efficiently and safely. Since the nucleic acid carrier guarantees a high level of gene transfer into the cells, it is useful for gene therapy.

## Claims

1. A nucleic acid carrier comprising a polypeptide comprising diaminobutyric acid and/or a pharmaceutically acceptable salt thereof and a recombinant viral vector.

2. The nucleic acid carrier according to claim 1, wherein the polypeptide comprises a block copolymer of a polypeptide comprising diaminobutyric acid and/or a pharmaceutically acceptable salt thereof and polyethylene glycol.

3. The nucleic acid carrier according to claim 1, wherein the peptide comprises diaminobutyric acid and/or a pharmaceutically acceptable salt thereof, each having a residue number of from 20 to 280.

4. The nucleic acid carrier according to claim 1, wherein the peptide comprises diaminobutyric acid having a residue number of from 20 to 280.

5. The nucleic acid carrier according to any of claims 1 to 4, wherein the recombinant viral vector comprises a therapeutic gene.

6. The nucleic acid carrier according to any of claims 1 to 4, wherein the recombinant viral vector is a HIV vector.

7. The nucleic acid carrier according to claim 5, wherein the recombinant viral vector is a HIV vector.

8. The nucleic acid carrier according to claim 6, wherein the HIV vector is a HIV vector of the psuedo-type with VSV-G.

9. The nucleic acid carrier according to claim 7, wherein the HIV vector is a HIV vector of the psuedo-type with VSV-G.

# Fig.1

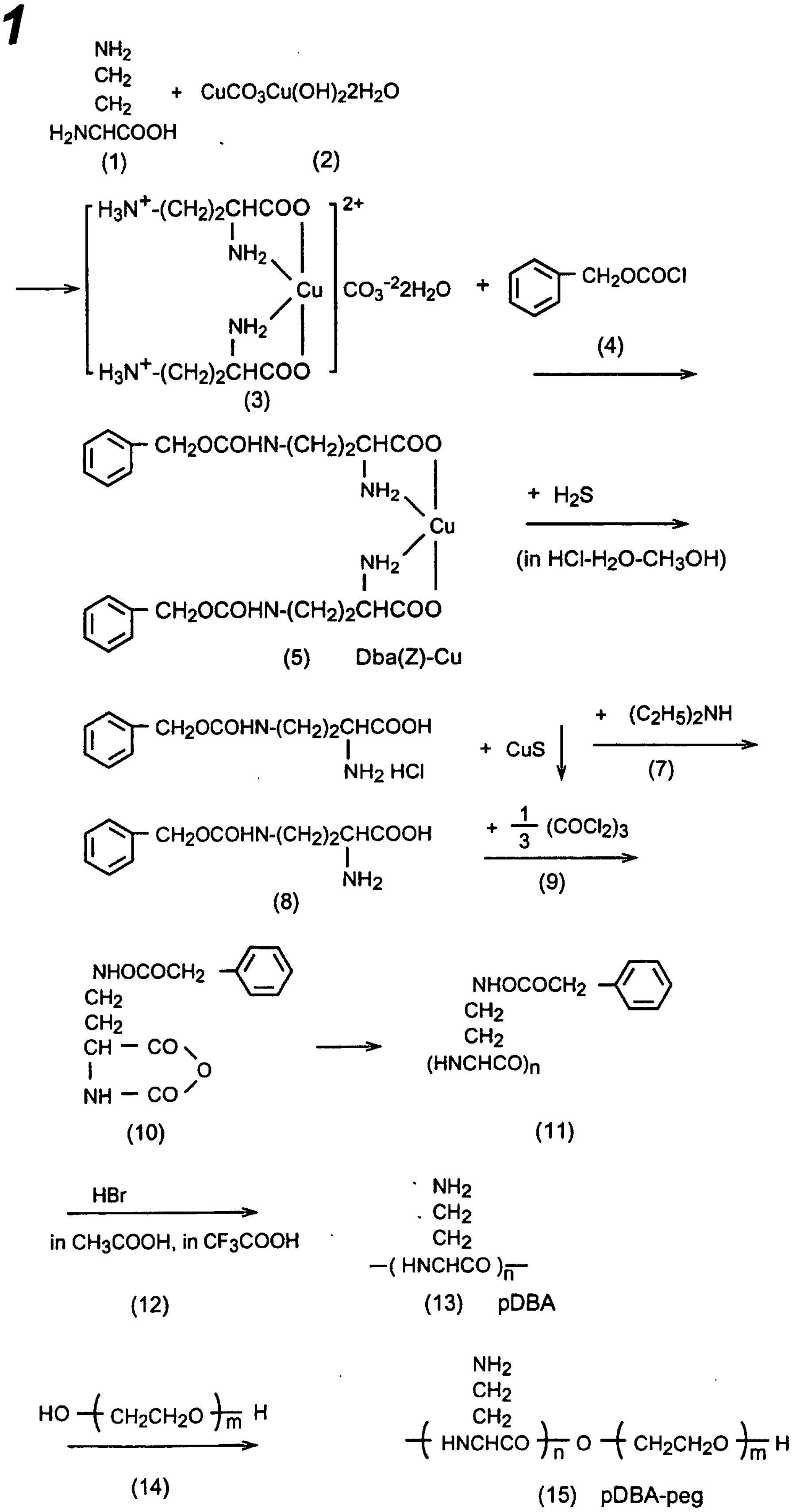
(1)
+ $CuCO_3Cu(OH)_22H_2O$
(2)
(3)
+
(4)
+ $H_2S$
(in $HCl$-$H_2O$-$CH_3OH$)
(5) Dba(Z)-Cu
+ CuS
+ $(C_2H_5)_2NH$
(7)
(8)
+ $\frac{1}{3}$ $(COCl_2)_3$
(9)
(10)
(11)
HBr
in $CH_3COOH$, in $CF_3COOH$
(12)
(13) pDBA
(14)
(15) pDBA-peg

*Fig.2*

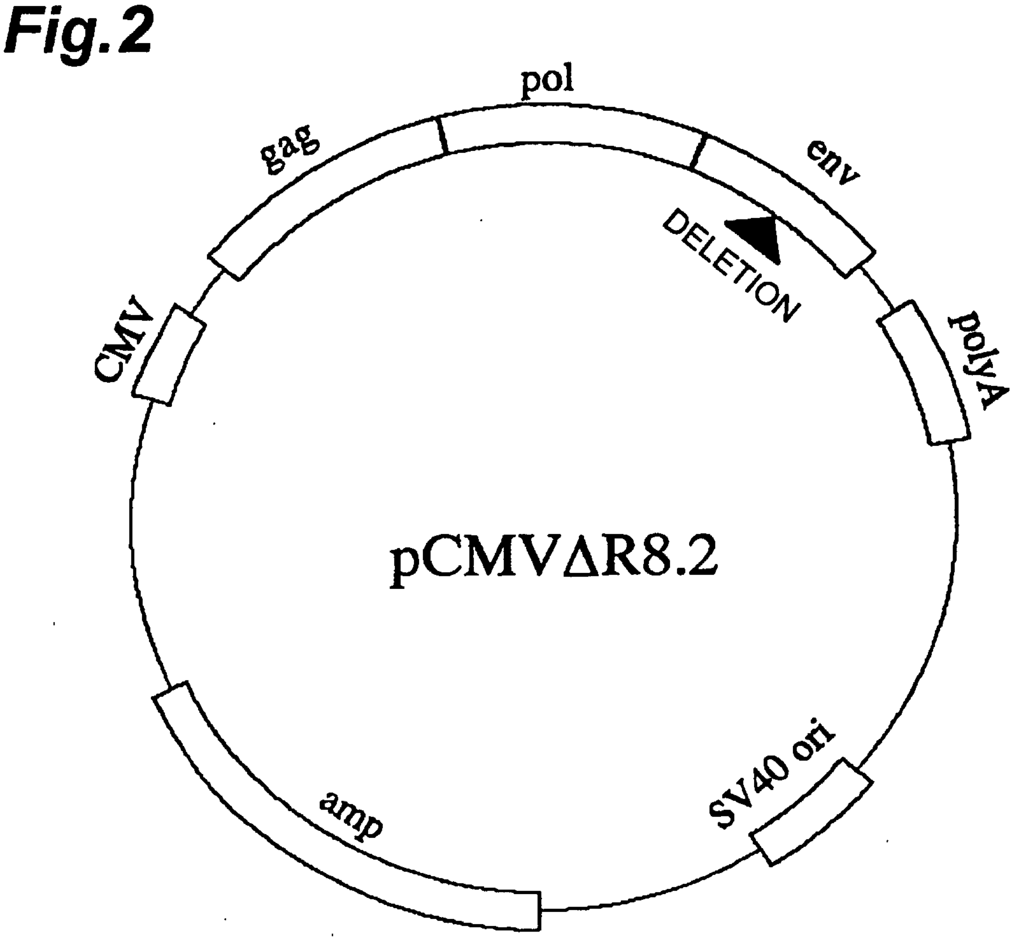

*Fig.3*

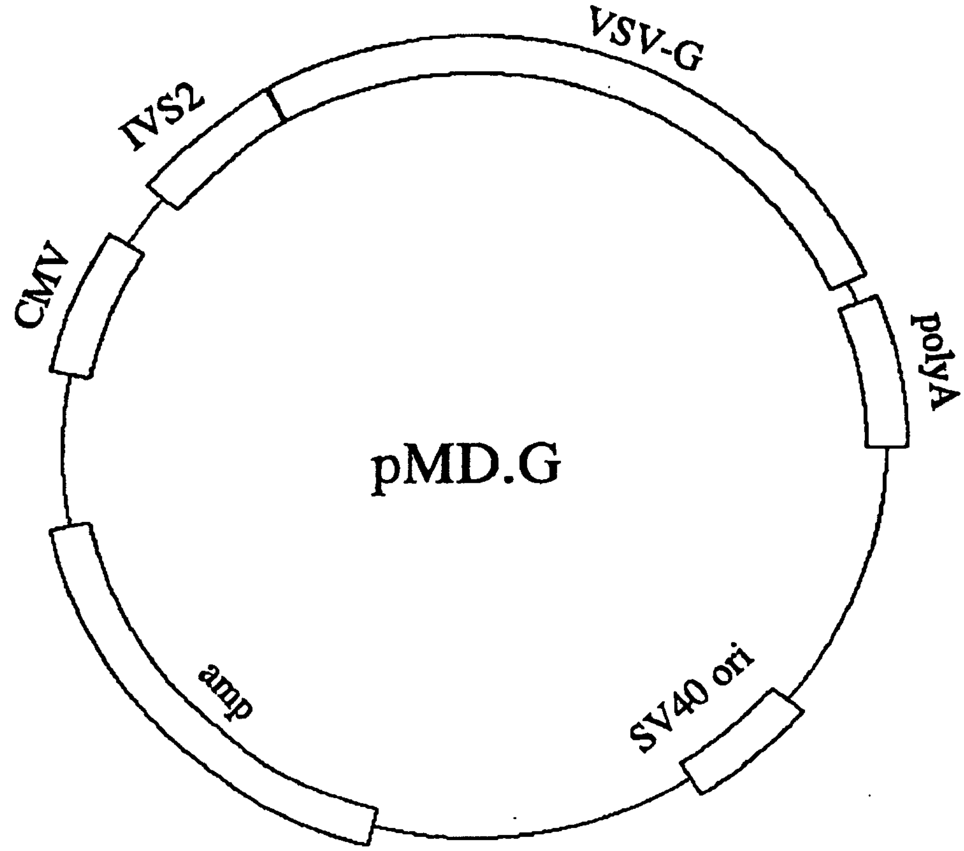

*Fig.4*

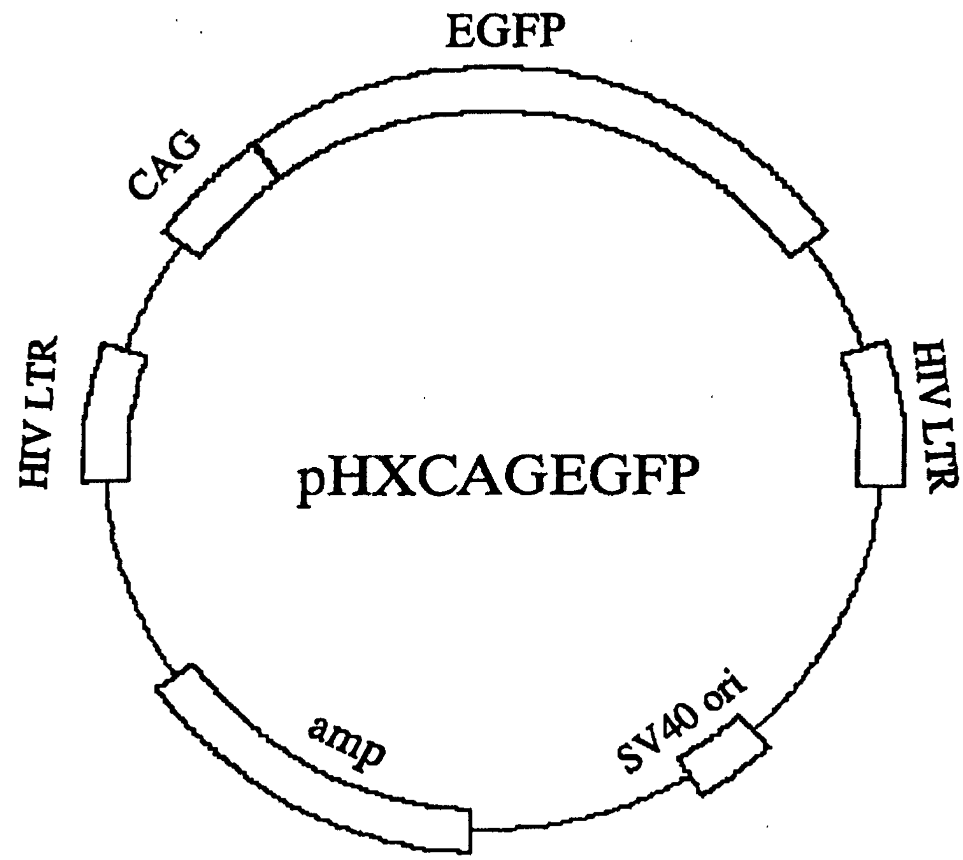

***Fig.5***

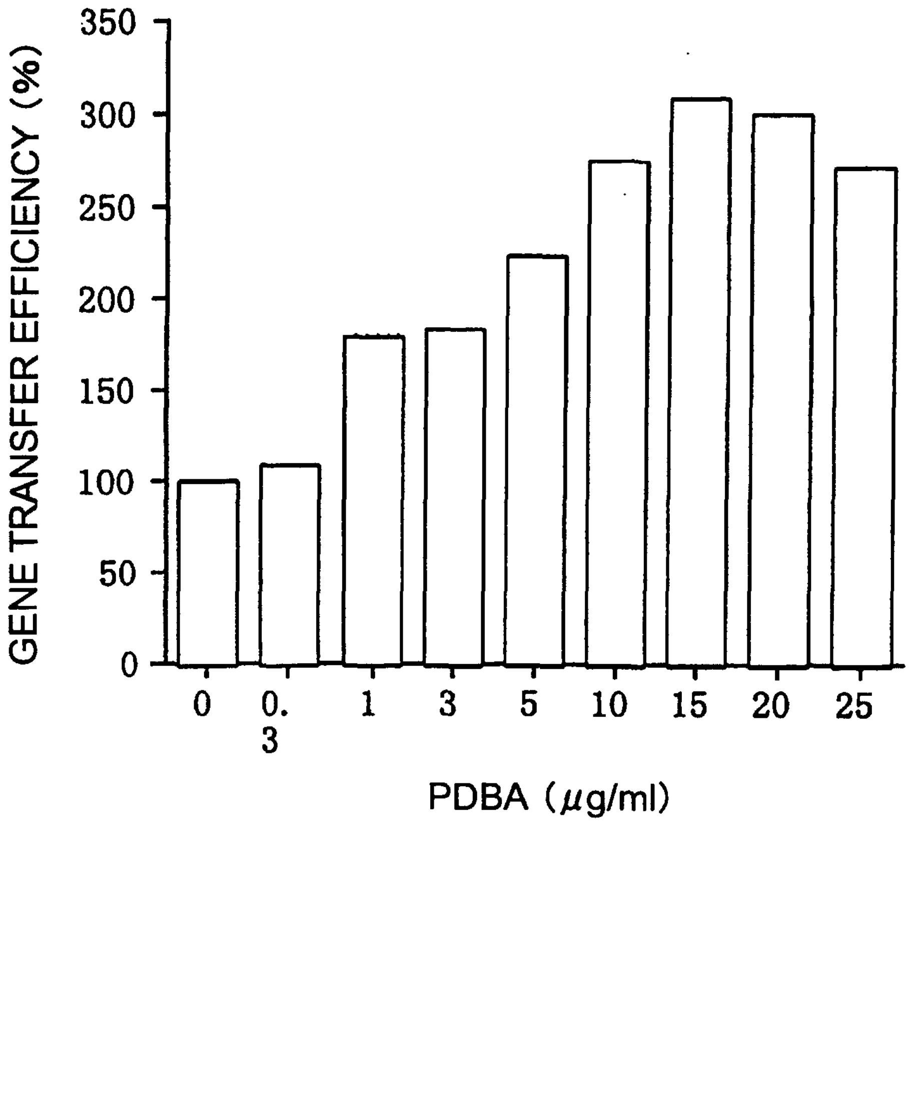
GENE TRANSFER EFFICIENCY (%)
350
300
250
200
150
100
50
0
0
0.3
1
3
5
10
15
20
25
PDBA (μg/ml)

*Fig.6*

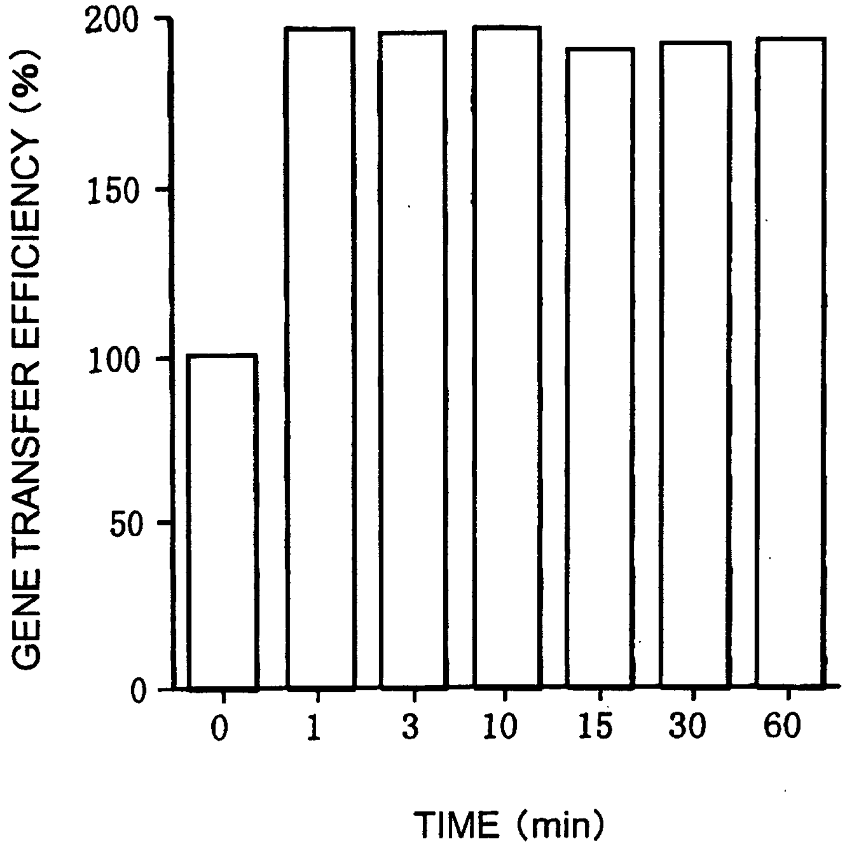
GENE TRANSFER EFFICIENCY (%)
200
150
100
50
0
0
1
3
10
15
30
60
TIME (min)

## INTERNATIONAL SEARCH REPORT

International application No.
PCT/JP00/07337

A. CLASSIFICATION OF SUBJECT MATTER
Int.Cl[7] C12N 15/867, C12N 15/90

According to International Patent Classification (IPC) or to both national classification and IPC

B. FIELDS SEARCHED
Minimum documentation searched (classification system followed by classification symbols)
Int.Cl[7] C12N 15/867, C12N 15/90

Documentation searched other than minimum documentation to the extent that such documents are included in the fields searched

Electronic data base consulted during the international search (name of data base and, where practicable, search terms used)
WPI, WPI/L, BIOSIS PREVIEWS, CAS ONLINE, GenBank/EMBL/DDBJ/Geneseq

C. DOCUMENTS CONSIDERED TO BE RELEVANT

| Category* | Citation of document, with indication, where appropriate, of the relevant passages | Relevant to claim No. |
|---|---|---|
| A | C.P.Hodgson et al., "Virosomes: Cationic Liposomes Enhance Retroviral Transduction" Nature Biotechnology, Vol. 14 (1996) pp.339-342 | 1-9 |
| A | D.T.Curiel et al., "Adenovirus enhancement of transferrin-polylysine-mediated gene delivery" Proc. Natl. Acad. Sci. U.S.A. Vol.88 (1991) pp.8850-8854 | 1-9 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 January, 2001 (16.01.01) | 13 February, 2001 (13.02.01) |
| Name and mailing address of the ISA/ Japanese Patent Office | Authorized officer |
| Facsimile No. | Telephone No. |

Form PCT/ISA/210 (second sheet) (July 1992)